# EUROPEAN PATENT APPLICATION

(11) **EP 1 408 048 A1**
(43) Date of publication of application: **14.04.2004**
(21) Application number: 02292473.2
(22) Date of filing: 07.10.2002
(51) Int. Cl.: C07K 7/06, C12N 15/11, A61K 39/00, A61K 31/711

(54) **Vaccines of enhanced immunogenicity, and methods for preparing such vaccines**

(71) Applicant: INSTITUT NATIONAL DE LA SANTE ET DE LA RECHERCHE MEDICALE (INSERM), 75013 Paris (FR)
(72) Inventor: Lavau, Grégoire, 06530 Peymeinade (FR); Firat, Huseyin, 68300 Saint Louis (FR); Saveanu, Loredana, 75015 Paris (FR); Lemonnier, François, 92340 Bourg la Reine (FR); Fruci, Doriana, 00124 Rome (IT); van Endert, Peter, 92160 Antony (FR)
(74) Representative: Bernasconi, Jean

(57) **Abstract**

The invention relates to an immunogenic or vaccine composition comprising a peptide, a polypeptide or a vector that expresses a peptide or polypeptide, wherein the peptide or polypeptide comprises one or several epitope(s), that are extended with one to three or four aminoacids at the N-terminus of the epitope(s).

The invention further relates to the preparation of this composition.

SEQUENCE LISTING

<110>  INSERM

<120>   Vaccines  of  enhanced  immunogenicity,  and  methods  for
preparing
        such vaccines

<130>  BET 02/0806

<140>  EP 02292473.2
<141>  2002-10-07

<160>  4

<170>  PatentIn version 3.2

<210>  1
<211>  10
<212>  PRT
<213>  Artificial

<220>
<223>  peptide

<400>  1

Phe Leu Pro Ser Asp Phe Phe Pro Ser Val
1               5                   10

<210>  2
<211>  9
<212>  PRT
<213>  Artificial

<220>
<223>  peptide

<400>  2

Arg Arg Tyr Asn Ala Ser Thr Glu Leu
1               5

<210>  3
<211>  9
<212>  PRT
<213>  Artificial

<220>
<223>  peptide

<400>  3

Ser Leu Tyr Asn Thr Val Ala Thr Leu
1               5

<210>  4
<211>  13
<212>  PRT
<213>  Artificial

<220>
<223>  peptide

<400>  4

Thr Pro Pro Ala Tyr Arg Pro Pro Asn Ala Pro Ile Leu
1               5                   10

## Description

The present invention relates to vaccines of enhanced immunogenicity, and methods for preparing such vaccines.

The understanding of the cellular presentation of vaccine antigens and their recognition by T lymphocytes represents the basis for rational contemporary approaches to vaccine design. Molecularly defined vaccines are safer than traditional attenuated or inactivated pathogens and can theoretically be formulated to achieve a desired immunity to specific pathogen components. Such vaccines can be composed either of proteins or peptides known to be recognized by lymphocytes. Peptide vaccination is specifically used where responses by cytotoxic T lymphocytes need to be primed. These responses are likely to be crucial for obtaining protective immunity against important diseases such as infection by HIV or hepatitis C virus, but also in approaches to tumour immunotherapy. As a consequence, peptide vaccines, composed of single or multiple peptides, the latter sometimes linked in synthetic "poly-epitopes", have been developed and evaluated by a large number of investigators.

While such vaccines hold substantial promise for priming of CTL responses, they must be designed to ensure access of sufficient peptide quantities to HLA class I molecules. For peptides delivered to the cytosol, this requires efficient peptide transport by the TAP transporters (for "Transporter associated with Antigen Processing") to the lumen of the endoplasmic reticulum (ER) where nascent class I molecules assemble with peptides generated in the cytosol.

Van Endert et al., 1995 showed that TAP selects peptides according to a binding motif based on the strong effects on peptide affinity of the three NH₂-terminal positions and the COOH-terminal residues.

Lauvau et al., 1999 further showed that TAP can influence epitope selection and restrict access to the endoplasmic reticulum to epitope precursors.

For poly-epitopes, an additional requirement is proteolytic cleavage of the poly-epitope to release the individual epitopes. In the cytosol, this cleavage is carried out by the proteasome, which is known to be responsible for essentially all cuts at the carboxyterminals (Ct) of antigenic peptides in the cytosol. Multiple so-called trimming peptidases can also act on HLA class I-presentable peptides. These enzymes can remove aminoterminal (Nt or N-term) residues either before or after peptide transport into the ER.

Fruci et al., 2001 studied class I-independent amino-terminal trimming of epitope precursor peptides in the ER. The authors more particularly showed that several precursor peptides with one aminoacid extension at the NH₂ terminus are rapidly converted to HLA-A2 ligands by metallopeptidases of endoplasmic reticulum.

The inventors have now designed short sequence tags that enhance the intracellular processing of peptides for presentation by HLA class I molecules. The principal effect of such sequences is enhancement of peptide transport from the cytoplasm to the lumen of the ER. Evidence is provided that several selected tags enhance this transport dramatically and for all tested peptides. Moreover, the inventors show directly that such tags can improve presentation of one HLA-A2 presented epitope significantly. The latter observation suggests that the Nt tags are efficiently removed by ER-luminal aminopeptidases. In addition to enhancing TAP transport, peptide tags are designed to direct proteasome cleavage in poly-epitopes so that cleavages within or directly after the tags are minimized.

A subject of the invention is thus an immunogenic or vaccine composition comprising a peptide, a polypeptide or a vector that expresses a peptide or polypeptide, in association with a pharmaceutically acceptable carrier, wherein the peptide or polypeptide comprises one or several epitope(s), that are extended with such short sequence tags, and are to be internalised by antigen presenting cells.

More particularly, the epitope is extended with one to three or four aminoacids at the N-terminus of the epitope so that the N-term sequence of the extended epitope is
X1-X2-X3 or X1-X2-X3-X4

Wherein
X1 is alanine, asparagine, arginine or lysine
X2 is arginine, glutamine, leucine or tryptophane
X3 is tyrosine, tryptophane, alanine or phenylalanine
X4 is any aminoacid.

The peptide or polypeptide so extended is also part of the present invention.

Another subject of the invention is a method for preparing this composition, which method comprises producing the peptide or polypeptide by chemical synthesis or by recombinant DNA techniques, for instance.

In a preferred embodiment, it is provided a polyepitopic vaccine, that consists of 2 to 20 or more HLA class I-binding epitopes linked to each other by means of the tags, specifically by tags resistant to internal or downstream proteasome cleavage. Polyepitopes can be expressed in DNA vaccines, but also synthesized and then coupled covalently to targeting agents, or produced in expression systems, possibly in the form of fusion proteins consisting of the poly-epitope coupled to the targeting agent.

The present invention thus relates to the use of a three or four aminoacid long extension inserted at the N-terminus of an epitope to be internalised by antigen presenting cells so that the N-term sequence of the extended epitope is X1-X2-X3 or X1-X2-X3-X4, wherein X1, X2, X3 and X4 are as above defined; for improving presentation of the epitope by the antigen presenting cells, in comparison with non-extended epitope, *i.e.* the epitope showing the same aminoacid sequence except for the N-terminal extension. In a particular aspect of the invention, the tag is inserted before the N-terminus of selected epitope(s) within the sequence of an antigenic protein, so as to obtain more efficient presentation of the peptides derived from proteasome degradation of the antigenic protein.

A further subject of the invention is a method for improving immunogenicity of a (poly)peptide by enhancing epitope presentation to antigen presenting cells, which method comprises inserting a three or four aminoacid long extension at the N-terminus of an epitope to be internalised by antigen presenting cells, so that the N-term sequence of the extended epitope is X1-X2-X3 or X1-X2-X3-X4, wherein X1, X2, X3 and X4 are as above defined.

### Definitions

In the context of the invention, "*peptides*" are two or more aminoacids joined by a peptide bond. Peptides can vary in length from dipeptides with two aminoacids to peptides with 10-20 aminoacids. Longer peptides, with up to several hundred aminoacids, are usually called "*polypeptides*" (Walker, Chamber Biological Dictionary, Cambridge, England : chambers Cambridge, 1989, page 215). Throughout the present text, the term (poly)peptide means a peptide or a polypeptide.

The peptides or polypeptides according to the present invention only comprise naturally occurring aminoacids.

In the context of the present invention, "*polypeptides*" include "polyproteins", that are made of individual proteins that are joined together in a sequence whereby they retain their original relevant biological activities.

An *"antigen"* refers to a molecule, such as a peptide, a polypeptide, or a protein against which an immune response is sought. Said antigen may be for instance a tumor antigen or an antigen of a pathogenic agent, such as a bacterial or viral antigen.

Various tumor antigens, including, for example, epithelial cell mucin, which is encoded by the MUC-1 gene, and the melanoma antigen MZ2-E, which is encoded by the MAGE-1 gene, are associated with particular tumor cells. Her2-neu is another exemplary tumor antigen associated with breast cancer. Additional tumor antigens include p100, NY-ESO, telomerase, and the proteins of the MAGE and BAGE families.

The viral or bacteria antigen may be of any origin. HIV or hepatitis C virus antigens are of particular interest.

An "*epitope*" is a fragment of the aminoacid sequence of the antigen that, upon binding to an HLA molecule, is recognized by a T cell receptor.

The epitopes as referred herein to are internalised by antigen presenting cells and presented at the surface of these cells after assembling with HLA class I molecules.

A "*native epitope*" is an epitope that naturally occurs in nature.

A "*polyepitope*" means a polypeptide or polyprotein which is made of individual epitopes joined together in a sequence. The epitopes of the polyepitope may be from the same origin, *i.e.* from the same antigen or from the same pathogenic agent, or may be from various antigens or pathogenic agents. Polyepitopic polypeptides of the invention also include native antigenic polypeptides or proteins that comprise several epitopes.

The term "*extended epitope*" refers to an epitope, the N-terminal aminoacid sequence of which has been extended by at least one extra aminoacid or a short sequence, also called *"extension sequence"* or "*tag*".

"*Antigen presenting cells*" as referred herein, express at least one MHC determinant and include those cells which are known as professional antigen-presenting cells such as macrophages, dendritic cells and B cells. Other professional antigen-presenting cells include monocytes, marginal zone Kupffer cells, microglia, Langherhans' cells, interdigitating dendritic cells, follicular dendritic cells, and T cells.

Unless otherwise specified, *"antigen presenting cells"* as referred herein, may also include "non professional" APC, *e.g.* activated T cells, astrocytes, follicular cells, endothelium and fibroblasts.

The terms "*vector*", "*cloning vector*" and "*expression vector*" mean the vehicle by which a DNA or RNA sequence (*e.g.* a foreign gene) can be introduced into a host cell, so as to transform the host and promote expression (*e.g.* transcription and translation) of the introduced sequence. Vectors include plasmids, phages, viruses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of doublestranded DNA, usually of bacterial origin, that can readily accept additional (foreign) DNA and which can be readily introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts. Non-limiting examples include pKK plasmids (Clontech), pUC plasmids, pET plasmids (Novagen, Inc., Madison, WI), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, *e.g.* antibiotic resistance, and one or more expression cassettes.

The terms "*express*" and "*expression*" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, *e.g.* the resulting protein, may also be said to be "expressed" by the cell. An expression product can be characterized as intracellular, extracellular or secreted. The term "*intracellular*" means something that is inside a cell. The term "*extracellular*" means something that is outside a cell. A substance is "secreted" by a cell if it appears in significant measure outside the cell, from somewhere on or inside the cell.

The term "*transfection*" means the introduction of a foreign nucleic acid into a cell. The term "*transformation*" means the introduction of a "foreign" (*i.e.* extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA bas been "transformed" and is a "transformant" or a "clone." The DNA or RNA introduced to a host cell can come from any source, including cells of the same genus or species as the host cell, or cells of a different genus or species.

The term "*host cell*" means any cell of any organism that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described *infra.*

The term "*expression system*" means a host cell and compatible vector under suitable conditions, *e.g.* for the expression of a protein coded for by foreign DNA carried by the vector and introduced to the host cell. Common expression systems include *E. coli* host cells and plasmid vectors, insect host cells and *Baculovirus* vectors, and mammalian host cells and vectors. Suitable cells include fibroblasts or lymphoblastoid B cell lines, as well as COS-1 or C₂C₁₂ cells, CHO cells, HeLa cells, 293T (human kidney cells), mouse primary myoblasts, and NIH 3T3 cells.

"*Pharmaceutically*" or "*pharmaceutically acceptable*" refer to molecular entities and compositions that do not produce an adverse, allergic or other untoward reaction when administered to an animal, or a human, as appropriate.

As used herein, "*pharmaceutically acceptable carrier*" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### Tags

In one embodiment, the immunologic or vaccine composition comprises a (poly)peptide or a vector that expresses the (poly)peptide, wherein the (poly)peptide comprises one or several epitopes that are extended with only one aminoacid X1 at the N-term.

This is the case when the N-term sequence of the unextended native epitope is already X2-X3 or X2-X3-X4.

When the first N-term aminoacids of the unextended native epitope are already X3 or X3-X4, it is then needed to add two aminoacids X1-X2 to the N-terminus.

However, according to a preferred embodiment, three or four aminoacids are added so that the N-term sequence of the extended epitope is X1-X2-X3 or X1-X2-X3-X4, wherein X1, X2, X3 and X4 are as defined above.

In a particular embodiment, the (poly)peptide comprises one epitope that is extended with three or four aminoacids at the N-terminus so that the N-term sequence of the extended epitope is X1-X2-X3 or X1-X2-X3-X4, wherein X1, X2, X3 and X4 are as defined in the present invention.

In a first aspect of the invention, the (poly)peptide comprises one epitope only. When the (poly)peptide is a native antigenic protein or polypeptide, the tag of the invention can be inserted at the N-terminus of the epitope within the native antigenic protein or polypeptide. As a result the presentation of the epitopic peptide derived from this protein or polypeptide, by antigen presenting cells, is improved. This insertion may be achieved by any technique, *e.g.* site-directed mutagenesis.

In a second aspect of the invention, the (poly)peptide comprises more than one epitope. In that case, all epitopes may be extended by one of the tag of the invention, or only some (*i*.*e*. one or more) epitopes can be extended, and some others can remain untouched.

The extended N-terminus of one epitope can be identical or different from the extended N-terminus of another epitope in the same polypeptide.

Preferably 2 to 20, more preferably 2 to 10, still more preferably 2 to 6 epitopes may be present in the polypeptide. Yet more than 20 epitopes may be extended similarly within the same polypeptide.

In a first embodiment, the epitopes in the polyepitopic polypeptide are linked to each other by one of the tags described above.

However in a second embodiment, the tags of the invention can also be used to modify the sequence of antigenic proteins that are to be degraded by the proteasome, so as to obtain more efficient presentation of peptides derived by proteasome processing. For that purpose the tags are introduced at the N-terminus of selected epitopes within the sequence of the antigenic protein. The introduction may be achieved by any technique, e.g. site-directed mutagenesis.

Polyepitopic vaccines so produced are particularly advantageous, since all the epitopes are simultaneously presented.

In a particular embodiment, the polypeptide comprises several epitopes extended with three or four aminoacids at the N-terminus so that the N-term sequence of the extended epitope is X1-X2-X3 or X1-X2-X3-X4, wherein X1, X2, X3 and X4 are as defined in the present invention.

Preferably, the tags, and specifically tags to be used in poly-epitopes, are designed to be resistant to internal cleavage by the proteasome in the cytosol. This means that they are designed to favor cleavage ahead of the tag while inhibiting cleavage within or immediately after the tag. Preferably when the polyepitope consists of a polyepitopic polypeptide wherein the epitopes are linked to each other by one of the tags described above, tags are therefore designed so that the polyepitope is to be cleaved after the C-terminus of each incorporated epitope. As a result N-terminally tagged individual epitopes are released for efficient transport into the endoplasmic reticulum.

For that purpose, it is preferred when X1 is N, X2 is W or Q and X3 is W. The tags are then NWW, NWWX4, NQW or NQWX4.

The N-term sequence of the extended epitope may also be selected from the group consisting of sequences :
- ARY
- NRY
- NQY
- and NRA.

Advantageously, all these specific tags can be added to a native epitope. When desired, X4 is chosen so as the full tag is still recognized by the peptidases that remove the tag before presentation of the epitope at the cell surface.

### Targeting agents

Peptide vaccines can be administered by injection of synthetic peptides or peptide mixtures. However, in the case of HLA class I-presented peptides, such peptides are as likely to be bound and presented by "non-professional" antigen presenting cells (APC), *i.e.* parenchymal cells, as by "professional" APC, *i.e.* dendritic cells and macrophages. Peptide presentation by the former APC, which lack co-stimulatory molecules, induces T cell anergy or tolerance while presentation by the latter can induce productive immune responses. As a result, synthetic peptide vaccines tend to induce a mixture of productive and abortive responses, so that strong adjuvants are required to shift the balance to the productive side.

Considering these facts, the presentation of vaccine peptides should ideally be restricted to professional APCs that will prime and expand but not tolerise CTLs. This can be achieved by targeting vaccines to the desired cell type, for example by coupling them to molecular devices or targeting agents such as antibodies to cell surface markers or heat shock proteins that are taken up by the cells.

The peptides or polypeptides designed above can be coupled to a targeting agent that addresses the (poly)peptide(s) to professional antigen presenting cells when the immunogenic or vaccine composition is administered *in vivo.* The targeting agent may be a protein that mediates specific vaccine binding to, and internalization by, a cell type of interest - in this case mainly dendritic cells. These targeting proteins generally bind to cell surface receptors and mediate the internalisation thereof. Dendritic cells express numerous cell surface receptors that, when targeted by specific antibodies, may be suitable for this purpose, for example Fc receptors, lectins such as DEC-205, mannose receptor or DC-SIGN, and scavenger receptors. Certain bacterial proteins, such as adenylate cyclase toxin of *Bordetella pertussis* (Guermonprez et al., 2001), are also internalized by dendritic cells and are therefore also suitable. Receptor/ligand internalization is followed by transfer of internalized antigens from endosomes to the cytosol of dendritic cells. This transfer has been described more particularly both for Fc-receptor and adenylate cyclase-mediated endocytosis (Guermonprez et al. 2002).

Vaccine peptides are then released within the professional APC and can assemble with HLA class I molecules subsequently exported to the cell surface. Since such vaccines are fed into the physiological antigen presentation pathways exclusively in professional APCs, they should be much more efficient than standard injectable synthetic peptides. Vaccine delivery to professional APCs also has the advantage that multiple peptides can be linked in polyepitope chains that can then be decomposed into individual epitopes within the APCs.

### Vectors

In one embodiment of the invention, the immunologic or vaccine composition comprises a peptide or polypeptide.

In another embodiment of the invention, the immunologic or vaccine composition comprises a vector that expresses a peptide or polypeptide.

The vector may be of DNA, *e.g.* a DNA plasmid.

DNA vectors can be introduced into the desired host cells by any methods known in the art.

Accordingly it is possible to introduce the DNA vector *in vivo* in a naked form, *i*.*e*. without the aid of any delivery vehicle or system that facilitates the transfection of the vector into cells (EP 465 529).

The patent applications WO-A-90 11 092, WO-A-93 19 183, WO-A-94 21 797 and WO-A-95 20 660 have proposed using the technique of polynucleotide vaccines. Different means of vaccination may also be used, as gene gun, *e.g.* by depositing DNA on the surface of gold particles and projecting it so as to penetrate into the skin of the subject (Tang et al., 1992), or injections by means of a liquid jet, which makes it possible to transfect, at one and the same time, skin, muscle, fatty tissues and mammary tissues (Furth et al., 1992; U.S. patent Nos 5,846,946; 5,620,896; 5,643,578; 5,580,589; 5,589,466; 5,693,622 and 5,703,055 ; Robinson et al., 1997 ; Luke et al., 1997 ; Norman et al., 1997 ; Bourne et al., 1996).

Microinjection, electroporation, calcium phosphate precipitation, charged lipids liposomal or nanocapsule formulations, and additional available techniques.

Nanocapsules can generally entrap compounds in a stable and reproducible way. To avoid side effects due to intracellular polymeric overloading, such ultrafine particles (sized around 0.1 µm) should be designed using polymers able to be degraded *in vivo*. Biodegradable polyalkylcyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

For the past decade, there has been increasing use of liposomes for encapsulation and transfection of nucleic acids *in vitro*. Synthetic cationic lipids designed to limit the difficulties and dangers encountered with liposome mediated transfection can be used to prepare liposomes for *in vivo* transfection of a gene encoding a marker. The use of cationic lipids may promote encapsulation of negatively charged nucleic acids, and also promote fusion with negatively charged cell membranes.

Alternatively, the vector may be in the form of a recombinant virus comprising, inserted into its genome, a nucleic acid sequence that encodes said epitopic peptide or polypeptide.

The viral vector may be preferably chosen from adenoviruses, retroviruses, especially lentivirus, as well as adeno-associated viruses (AAV), herpes virus, cytomegalovirus (CMV), vaccinia virus and the like. Vectors derived from adenoviruses, retroviruses or AAVs, HIV-derived retroviral vectors, incorporating heterologous nucleic acid sequences have been described in the literature (Akli et al., (1993); Stratford-Perricaudet et al. (1990) ; EP 185 573, Levrero et al. (1991) ; Le Gal la Salle et al. (1993) ; Roemer et al. (1992) ; Dobson et al. (1990) ; Chiocca et al. (1990) ; Miyanohara et al. (1992) ; WO 91/18088).

Lentivirus vectors are described in Firat et al, 2002.

Advantageously, the recombinant virus according to the invention is a defective virus. The term "*defective virus*" designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the nucleic acid that encodes the epitopic peptide or polypeptide of interest. Preferably, the defective virus nevertheless conserves the sequences of its genome which are necessary for the encapsulation of the viral particles.

It is particularly advantageous to use the nucleic acid sequences of the invention in a form incorporated in an adenovirus, an AAV or a defective recombinant retrovirus, such as a lentivirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA.

The defective recombinant viruses of the invention can be prepared by homologous recombination between a defective virus and a plasmid carrying, *inter alia,* the encoding sequence that encodes the epitopic peptide or polypeptide of interest (Levrero et al. (1991); Graham, (1984)). The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 (Graham et al. (1977)) which contains especially, integrated into its genome, the left part of the genome of an Ad5 adenovirus (12%). As example of a line which can be used for the preparation of defective recombinant retroviruses, there may be mentioned the CRIP line (Danos et al. (1988)). Alternative vectors, such as shuttle vectors, can also be used that permit the cloning of the desired gene in the vector backbone.

Then the viruses which have multiplied are recovered and purified according to conventional molecular biology techniques.

Targeted gene delivery is described in International Pat. Publication WO 95/28494, published October 1995.

Alternatively, the vector can be introduced *in vivo* by lipofection.

### Production of the (poly)peptide

The (poly)peptide that comprises the extended epitope(s) according to the invention can be produced by any standard technique well-known by the skilled person.

In one embodiment, the (poly)peptide is produced by chemical synthesis, either *de novo* or by adding the N-term tag of interest to an epitopic aminoacid sequence already available or produced.

(Poly)peptides can be synthesized using well-known solid phase method (Merrifield et al., 1962 and 1963 ; Mitchell et al., 1976 ; Tam et al. 1983), preferably using a commercially available peptide synthesis apparatus (such as that made by Applied Biosystems, Foster City, California) and following the manufacturer's instructions.

Alternatively, such peptides can be synthesized by recombinant DNA techniques as is now well-known in the art (Maniatis et al., 1982). For example, these peptides can be obtained as DNA expression products after incorporation of DNA sequences encoding the desired (poly)peptide isolated from the desired species into expression vectors and introduction of such vectors into suitable eukaryotic or prokaryotic hosts that will express the desired peptides individually or as part of fusion peptides or proteins, from which they can be later isolated using well-known techniques.

When introduction of tags within an antigenic protein is desired, any technique known in the art can be used, including but not limited to, in vitro site-directed mutagenesis (Kunkel et al, 1987), preferably using PCR techniques.

### Immunologic or vaccine compositions

The present invention further encompasses preparing immunogenic or vaccine compositions.

The compositions of the invention can be formulated for a intramuscular, oral, parenteral, intranasal, intravenous, subcutaneous, mucosal or intraperitoneal administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

When the immunogenic or vaccine composition comprises nucleic acids, the doses of nucleic acids (sequence or vector) used for the administration can be adapted as a function of various parameters, and in particular as a function of the mode of administration used, of the relevant pathology, of the nucleic acid to be expressed, or alternatively of the desired duration of treatment. Generally, with regard to recombinant viruses, the latter are formulated and administered in the form of doses of between 10⁴ and 10¹⁴ pfu/ml, and preferably 10⁶ to 10¹⁰ pfu/ml. The term pfu ("plaque forming unit") corresponds to the infectivity of a virus solution, and is determined by infecting an appropriate cell culture and measuring, generally after 48 hours, the number of plaques of infected cells. The techniques for determining the pfu titer of a viral solution are well documented in the literature.

To prepare compositions comprising a peptide or (poly)peptide, an effective amount of the (poly)peptide may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Although not absolutely requested, adjuvants that enhance the immune response can also be added. A number of adjuvants are known to those skilled in the art. Examples of suitable adjuvants include, for example, aluminium hydroxide ; Saponin ; detergents such as Tween 80 ; animal, mineral or vegetable oils, Corynebacterium or Propionibacterium -derived adjuvants ; Mycobacterium bovis (Bacillus Calmette and Guerinn, or BCG) ; cytokines ; acrylic acid polymers such as carbomer ; EMA ; or combinations thereof.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15^{th} Edition, pages 1035-1038 and 1570-1580). Some variation in dosage will necessarily occur depending on the condition of the subject being treated. The person responsible for administration will, in any event, determine the appropriate dose for the individual subject.

The peptide or polypeptide protein may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the parenteral administration, such as intravenous or intramuscular injection, other pharmaceutically acceptable forms include, e.g. tablets or other solids for oral administration.

At last the immunogenic or vaccine compositions of the invention may be monovalent, i.e. the epitopes they contain are from a sole pathogenic agent or tumor type, or plurivalent, i.e. the epitopes they contain are from various pathogenic agents or tumor types.

Therefore the immunogenic or vaccine compositions of the invention may comprise one or several peptides or polypeptides.

The compositions of the invention are useful to enhance immunogenicity of antigens, in the prospect of prophylactic or therapeutic vaccination.

A further subject of the invention is thus the use of a peptide or polypeptide or a vector that expresses a peptide or polypeptide, as above defined, for the preparation of a pharmaceutical composition for vaccination.

The subject to be treated may be any vertebrate, especially mammals, preferably humans, but also non-human animals, such as rodents, cattle, horses, sheep, goats, monkeys, cats and dogs.

Another subject of the invention is a method for vaccinating a subject, which method comprises administering to the subject in need of such treatment a therapeutically efficient amount of an immunogenic or vaccine composition as above defined.

The figures and examples illustrate the invention without limiting its scope.

### LEGENDS OF THE FIGURES

Figure 1 shows TAP affinities of variants of peptide FLPSDFFPSV (called F10V), SEQ ID N° 1, extended at the N-terminus as shown on the left hand side. TAP affinities were determined in a competitive binding assay. IC₅₀ values for competitor test peptides were normalized with respect to the IC₅₀ of reporter peptide RRYNASTEL (SEQ ID N° 2).

Figure 2 shows TAP affinities of Her2-neu epitopes 48, 435 and 689 and of variants extended by the tag ARYS, measured and expressed as in Figure 1.

Figure 3 shows TAP affinities of Her2-neu epitopes.

Figure 4 shows effect of ARYX tags on presentation of epitope F10V to specific cytotoxic T lymphocytes. Lysis data obtained in cytotoxicity assays were corrected with respect to the cytosolic concentration of peptide in GFP⁺ cells.

### EXAMPLES

### EXAMPLE 1 : Effect of sequence tags on TAP affinity

### Materials and Methods

**Peptides.** Peptides F10V (FLPSDFFPSV) of SEQ ID N° 1, R9L of SEQ ID N°2 (RRYNASTEL) and S9L of SEQ ID N°3 (SLYNTVATL) were purchased from Genosys (Cambridge, U.K.) 95% pure. Precursors of F10V extended by one to three residues were synthesized with free amino acid termini using an Applied Biosystem SYNERGY peptide synthesizer and Fmoc chemistry. Purity was controlled by analytical reverse-phase chromatography on a Vydac C18 column and, when necessary, synthetic peptides were purified by reverse-phase chromatography to >95% purity. Sequence and purity were controlled also by mass spectrometry.

**Determination of HLA-A2 and TAP affinities.** TAP and HLA-A2 affinities were measured in competitive binding assays with radio-labeled reporter peptides R9L and F10V (6Y) used at 300 nM and 170 nM, respectively, as described before (Lauvau et al., 1999). Results are expressed as inverse normalized IC₅₀ (1/IC₅₀ of test peptide/ IC₅₀ of cold reporter peptide).

### Results

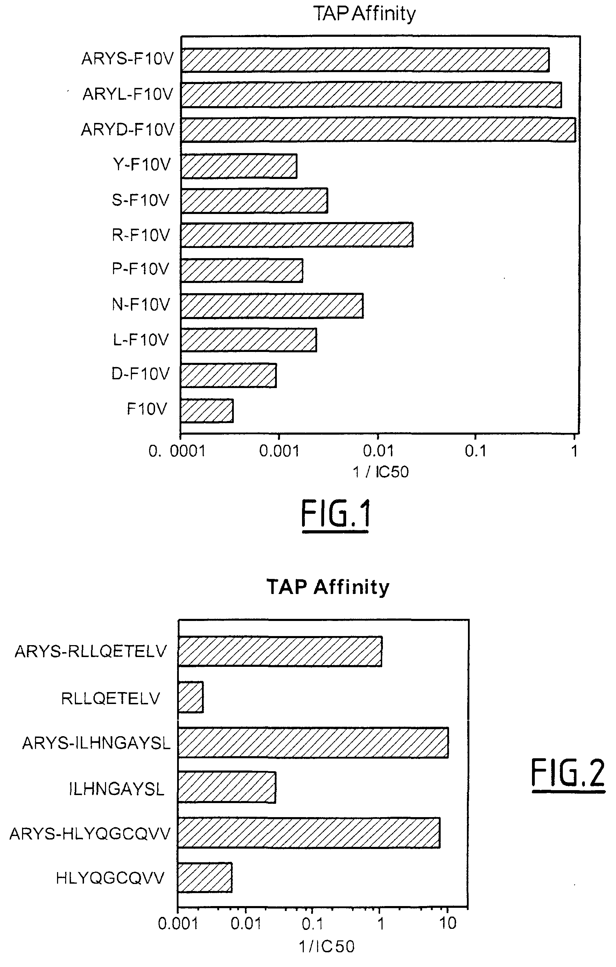

A first group of experiments was designed to establish whether defined 3- or 4-residue sequences are able to increase TAP transporter affinities of multiple peptides consistently, as shown in figures 1 and 2. Figure 1 demonstrates that addition of the Nt tag ARY (Ala-Arg-Tyr) increases "TAP affinity" (*i*.*e*. the inverse of the IC₅₀) of the peptide F10V by more than three logs. Peptide F10V, an HLA-A2 ligand, has a very low TAP affinity so that it could initially not be ruled out that the effect of the ARY tag will not extend to peptides with higher TAP affinities (but see below). However, it should be noted that ARY-tagged F10V variants have an inverse IC₅₀ close to 1, *i*.*e*. similar to that of the reporter peptide R9L, a peptide with high TAP affinity. Thus, the ARY tag increases TAP affinity from extremely low to high values. Addition of single residues to peptide F10V increases TAP affinity to a variable extent but less than the ARY tag. Finally, although addition of Ser, Leu or Asp (S,D,L) alone has distinct effects on TAP affinity, these residues do not affect TAP affinity when preceded by the ARY tag, confirming that TAP affinity depends only on the 3 Nt and the Ct residue.

To establish whether the effect of the ARY tag extended to other peptide epitopes and especially to those with higher TAP affinities, the inventors studied 3 additional HLA-A2 restricted epitopes derived from the tumour antigen Her-2neu (Figure 2). Importantly, all three epitopes had higher TAP affinities than peptide F10V, specifically peptide 435 with an intermediate affinity. Like for peptide F10V, addition of the tag consistently increased TAP affinity by more than two logs. Tagged peptides 435 and 689 even attained very high TAP affinities equalling the highest values measured so far by the inventors. Thus, the ARY tag induces a uniform and substantial increase in TAP affinity in peptides with very low to intermediate TAP affinities. The inventors have previously shown that such peptides include the vast majority of HLA class I ligands (Daniel et al, 1998).

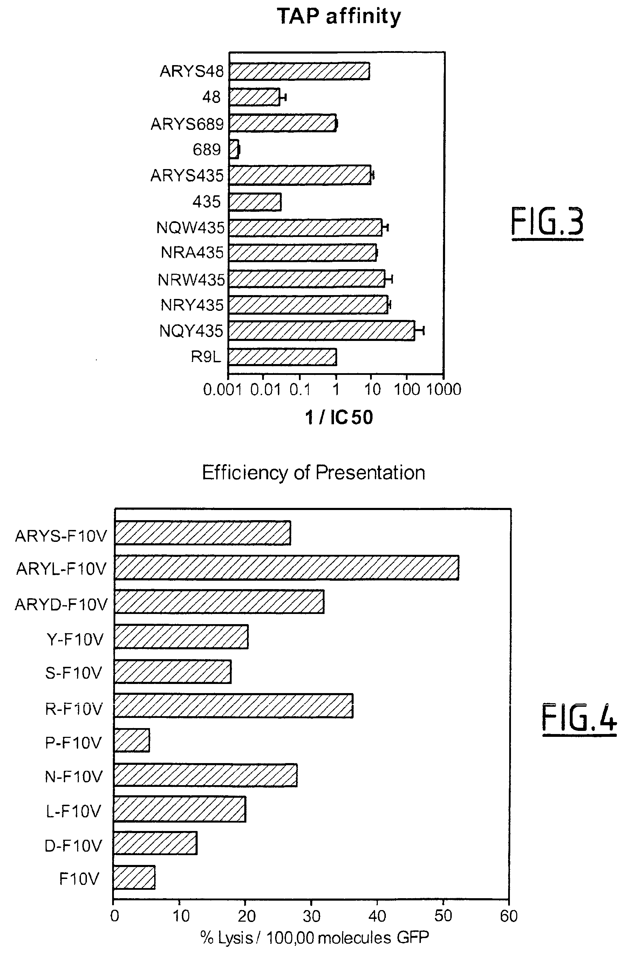

The ARY tag had been designed to increase TAP affinity but does not take proteasome digestion of poly-epitopes linked by tags into account. Both Arg and Tyr represent preferred proteasome cleavage sites, and even cleavages after Ala are not rare. Therefore the inventors designed additional tags expected to be more resistant to proteasome cleavage. Figure 3 shows that proteasome-resistant tags also conferred very high TAP affinities to variants of epitope Her2-neu 435. in fact, peptide NQY435 has the highest TAP affinity. The figure demonstrates that stepwise replacement of proteasome-susceptible residues (Ala, Arg and Tyr) by resistant ones (Asn, Gln, Trp) is entirely compatible with very high TAP affinities. Therefore, Nt sequence tags can be designed that are likely to resist proteasome attack while conferring very high transport efficiency.

These data show that TAP affinity can consistently be increased by short Nt sequence tags that may also be able to direct proteasome cleavage of poly-epitopes.

### Example 2 : Effect of sequence tags on peptide presentation to cytotoxic T cells

### Materials and methods

**Expression system.** An inducible system for coupled expression of peptides and a reporter protein was constructed by inserting an eGFP/ubiquitin cassette into the tetracycline-regulated expression plasmid MS4A. MS4A contains sequences encoding hygromycin resistance and the reverse tetracylin repressor-transactivator protein and a synthetic EBV-LMP2A promoter, in which the EBNA2 response element is substituted by a heptamerized *tet* operator sequence (Feuillard et al., 2000). MS4A was digested with Sfil, and complementary oligonucleotides encoding (5' to 3') Sfil, Nhel, Notl, Pacl and Sfil restriction sites were inserted. In parallel, a sequence encoding a fusion protein consisting of eGFP fused to ubiquitin with mutation Lys48Arg was amplified from plasmid peGFP/Ub (Valmori et al., 1999) with primers including a Nhel site (5') and a Notl site (3'). The resulting eGFP-Ub cassette was transferred as Nhel/Notl fragment into the modified MS4A vector, resulting in plasmid MS4A-eGFP/Ub. To insert peptide-encoding sequences, this plasmid was digested with Not I, followed by removal of protruding 5' overhangs with mung bean nuclease (1.5 U/µg, 3 min at 37°C), and finally digested with Pacl. Complementary oligonucleotides encoding peptides were designed to form blunt 5' ends upon hybridization and contained a TAG Stop codon and Pacl-complementary overhang at the 3' end. In the resulting constructs, the first codon of peptides was directly preceded by the terminal Gly codon of ubiquitin. Correct sequences of the eGFP/Ub cassette and of peptide-encoding sequences were verified by sequencing. A construct with a single nucleotide deletion in the sequence encoding R-F10V was used as negative control in some experiments.

**Transfectants.** To study CTL recognition of synthetic peptides, murine TAP2-deficient RMA-S (Pascolo et al., 1997) or HLA class I-deficient human lymphoblastoid C1R (Storkus et al., 1989) cells transfected with the class I molecule "HHD" were used. HHD consists of human β₂m linked to the aminoterminus of a chimeric class I heavy chain composed of the HLA-A*0201 α1 and α2 domains and the α3/transmembrane/cytosolic domains of H-2D^{b}. C1R-HHD transfectants were produced by electroporation of plasmid pBSK/HHD/neo; this plasmid is based on the published HHD expression plasmid (Pascolo et al., 1997) but contains the gene encoding neomycin resistance inserted as Sal I fragment. HHD-expressing cells were selected with 0.5 mg/ml G418 (Life Technologies). C1R-HHD transfectants expressed 65% of W6/32-reactive cell surface HLA class I molecules expressed by the homozygous human B cell line Jesthom (HLA-A2, B27): mean fluorescence channel 300 for C1R-HHD, 450 for Jesthom, 3 for second antibody only. Expression of eGFP/Ub/peptide fusion proteins did not affect HHD expression by C1R-HHD cells.

eGFP/Ub/peptide constructs were expressed in C1R-HHD cells. 20 µg plasmid was electroporated (250V and 960 µF) into 10⁷ cells suspended in 0.25 ml PBS with 10 mM Hepes (pH 7.4). 48 hours after transfection, selection of transfectants with 0.5 mg/ml hygromycin (Life Technologies) was initiated. Transfectants were used for analysis by fluorometry and kill assays between 10 and 30 days after transfection. eGFP expression decreased steadily over time so that cells were discarded 4 weeks after transfection. Experiments were performed with 3 independently produced sets of transfectants.

**Quantification of eGFP expression.** Expression of eGFP/Ub/peptide was induced by addition of doxycylin (Sigma) to cells cultured at 5x10⁵/ml 20 to 24 hours prior to experiments; G418 and hygromycin were omitted from the induction medium. Based on preliminary experiments, doxycylin concentrations of 0, 0.02, 0.08 and 0.6 µg/ml were used. eGFP expression was quantified both by flow cytometry and fluorometric determination of eGFP concentrations in cell lysates. For FACS analysis, cells were washed in PBS with 0.1 % NaN₃ and 1% FCS and re-suspended in the same buffer with 1 µg/ml propidium iodide (Sigma). eGFP expression by live cells excluding propidium iodide was then measured on a FACS Calibur™ machine. For fluorometric assays, cells washed in PBS were lysed for 30 min at 4°C in PBS with 1% NP-40, debris was removed by centrifugation at 20,000 x g for 5 min. Then cleared lysates were transferred into flat bottom 96 well plates (Maxisorb™, Nunc) and fluorescent light emitted at 510 nM was measured in a Cytofluorometer 2350 (Millipore) or a Jasco FP-750 fluorometer. To establish a calibration curve and calculate the eGFP concentration in transfectant lysates, an NP-40 lysate of untransfected C1R-HHD cells was used to prepare a series of dilutions of recombinant eGFP (Clontech). Using the molecular weight of eGFP and Avogadro's number, the number of eGFP molecules in the lysate was calculated. Finally, based on the number of cells lysed and the percentage of eGFP⁺ cells in the FACS analysis, the number of eGFP molecules per cell was determined.

**Cytotoxicity assays.** Cytotoxic T cells were generated by peptide immunization of HHD mice, which express the single chain chimeric HLA-A2/D^{b} molecule described above on a D^{b} and mouse β₂m knock out background. Mice were injected s.c. at the base of the tail with 50 µg of peptide F10V mixed with 140 µg helper peptide HBVcore 128-140 (TPPAYRPPNAPIL of SEQ ID N° 4) and emulsified in incomplete Freund's adjuvant (Difco, Detroit, MI). 11 days later, spleen cells were prepared and re-stimulated with irradiated, peptide-pulsed (5 x 10⁶ cells/ml, 10 µg/ml peptide, 2 hours at room temperature in FCS-free RPMI medium) and LPS-induced HHD lymphoblasts. Cultured CTL lines were used on days 5 or 6 after re-stimulation in 4-hour ⁵¹Cr-release assays. Specific lysis was calculated as follows: (experimental release - spontaneous release)/(total release - spontaneous release) x 100. Transfected target cells were analyzed for eGFP expression by flow cytometry on the day of the kill assay, and by fluorometric determination of eGFP concentration subsequently; transfectant viability was in all cases > 90%.

**Data processing.** Data obtained in kill assays with eGFP/Ub/peptide transfectants were processed to allow for comparison. Firstly, data were adjusted as follows for % eGFP⁺ cells (as determined by FACS analysis): % lysis / % positive x 100. Corrected lysis data were then divided by the number of eGFP molecules per cell (expressed as multiples of 100,000) determined for the same transfectants by fluorometric assay. Data obtained were analyzed by non-linear regression using Prism Graph™ software, and regression curves were used to determine lysis at 150,000 molecules eGFP/cell.

### Results

The inventors further studied the effect of Nt tags on peptide presentation to cytotoxic T cells. This was required since sequence tags reduce HLA class I binding affinity (not shown) and must therefore be removed to allow HLA class I presentation. The results are shown in Figure 4.

The figure demonstrates that all ARYX-tagged F10V variants are presented with high efficiency to CTL. However, the relative efficiency is highest when X is Leu. Since residue 4 of the tags does not affect TAP affinity, this may suggest that Leu is a more efficient substrate for luminal peptidases than Ser or Asp. Importantly, all 4-residue tags conferred high presentation efficiency that equalled that of the best presented peptide with a single residue extension. Thus, Nt sequence tags not only increase TAP affinity significantly, but can also be removed by luminal peptidases with sufficient efficiency to allow high level presentation of trimmed epitopes.

Similar studies are performed for the 3 Her2-neu epitopes, and for "proteasome-resistant" tags.

For that purpose, the three Her2-neu epitopes are cloned into an expression vector (pGeneSwitch, Invitrogen). The epitopes are expressed with or without various aminoterminal extensions, by transfecting C1 R-H4D cells.

### Example 3 : Effect of tags on the protective potential of epitope or polyepitope vaccines

The inventors are evaluating the effect of sequence tags on the protective potential of epitope or polyepitope vaccines. For that purpose the extended or non-extended epitopes are expressed in recombinant lentivirus (Firat et al., 2002). The viral vector is then used to immunize HLA-2 transgenic mice.

This vaccination is followed by challenge with Her2-neu expressing recombinant vaccine virus that can cause a severe respiratory infection in mice.

### REFERENCES

Akli et al. (1993) Nature Genetics 3:224
Bourne et al., (1996) J. Infec. Dis. 173 :8
Chiocca et al. (1990) New Biol. 2:739
Daniel et al, (1998), J. Immunol. 161: 617-24
Danos et al. (1988) PNAS 85:6460
Dobson et al. (1990) Neuron 5 :353
Firat et al., (2002), J. Gen. Ther. 4:38-45
Feuillard et al. (2000) *Blood 95:2068*
Fruci et al., (2001) Immunity 15 :467-476
Furth et al., (1992) analytical Biochemistry, 205:365-368
Graham (1984) EMBO J. 12:2917
Graham et al. (1977) J. Gen. Virol. 36: 59
Guermonprez et al., (2001) J. Exp. Med., 193(9) :1035-1044
Guermonprez et al. (2002) Annu Rev Immunol 20 :621-67
Kunkel et al, (1987) Methods in Enzymol., 154:367-382
Lauvau et al., (1999) J Exp Med *190:1227*
Le Gal la Salle et al. (1993) Science 259 988
Levrero et al., (1991) Gene 101 :195
Luke et al., (1997) J. Infect. Dis. 175(1):91-97
Maniatis et al. (1982) Molecular cloning : a laboratory manual, Cold Spring Harbor Laboratories, NY, 51-54 and 412-30
Merrifield, (1962) Proc. Soc. Ex. Boil. 21:412
Merrifield, (1963) J. Am. Chem. Soc. 85:2149
Mitchell et al. (1976) J. Am. Chem. Soc. 98:7357
Miyanohara et al., (1992) New Biol. 4:238
Norman et al., (1997) Vaccine, 15(8) :801-803
Pascolo et al. (1997) J Exp Med 185:2043.
Robinson et al., (1997) Immuology 9 :271-83
Roemer et al. (1992) Eur. J. Biochem. 208 211
Storkus et al. (1989) Proc Natl Acad Sci U S A 86:2361
Stratford-Perricaudet et al. (1990) Human Gene Therapy 1:241
Tam et al., (1983) J. Am. Chem. Soc. 105:6442
Tang et al., (1992) Nature 356, 152-154
Valmori et al. ( 1999) J Exp Med 189:895
Van Endert et al., (1995) J. Exp. Med. 182:1883-1895

## Claims

1. A (poly)peptide, which comprises one or several epitope(s), that are extended with one to three or four aminoacids at the N-terminus of the epitope(s) so that the N-term sequence of the extended epitope(s) is X1-X2-X3 or X1-X2-X3-X4,
wherein X1 is alanine, asparagine, arginine or lysine;
X2 is arginine, glutamine, leucine or tryptophane;
X3 is tyrosine, tryptophane, alanine or phenylalanine;
X4 is any aminoacid;
and wherein the epitopes are to be internalised by antigen presenting cells.

2. The (poly)peptide of claim 1, which comprises one epitope that is extended with three or four aminoacids at the N-terminus so that the N-term sequence of the extended epitope is X1-X2-X3 or X1-X2-X3-X4, wherein X1, X2, X3 and X4 are as defined in claim 1.

3. The (poly)peptide of claim 1, which comprises several epitopes, each being extended with one to three or four aminoacids at the N-terminus of the epitope(s) so that the N-term sequence of the extended epitope(s) is X1-X2-X3 or X1-X2-X3-X4, wherein X1, X2, X3 and X4 are as defined in claim 1.

4. The (poly)peptide of claim 3, which comprises several epitopes, each being with three or four aminoacids at the N-terminus so that the N-term sequence of each extended epitope is X1-X2-X3 or X1-X2-X3-X4, wherein X1, X2, X3 and X4 are as defined in claim 1.

5. The (poly)peptide according to any of claims 1, 3 or 4, which is a polyepitopic polypeptide comprising several epitopes that are linked to each other by one to three or four aminoacids so that the N-term sequence of the extended epitope(s) is X1-X2-X3 or X1-X2-X3-X4, wherein X1, X2, X3 or X4, are as defined in claim 1.

6. The (poly)peptide according to any of claims 1 to 5, wherein the N-term sequence of the extended epitope is selected from the group consisting of sequences:
- ARY
- NRY
- NQY
- NRW
- NWW
- and NRA.

7. The (poly)peptide according to claim 6, wherein the N-term sequence has been added to a native epitope.

8. A vector that expresses the (poly)peptide according to any of claims 1 to 7.

9. An immunogenic or vaccine composition comprising a peptide, a polypeptide or a vector that expresses a peptide or polypeptide, in association with a pharmaceutically acceptable carrier, wherein the peptide or polypeptide is as defined in any of claims 1 to 7.

10. The composition according to claim 9, comprising a peptide or polypeptide coupled to a targeting agent, wherein the targeting agent addresses the peptide or polypeptide to an antigen presenting cell when the composition is administered *in vivo*.

11. The composition according to claim 9, comprising a vector that expresses said peptide or polypeptide, wherein said vector is DNA.

12. The composition according to claim 9, comprising a vector that expresses said peptide or polypeptide, wherein said vector is a recombinant virus.

13. A method for preparing the composition according to claim 9, which method comprises producing the peptide or polypeptide by chemical synthesis.

14. A method for preparing the composition according to claim 9, which method comprises producing the peptide or polypeptide by expression of a DNA that encodes said peptide or polypeptide in a host cell.

15. Use of a three or four aminoacid long extension inserted at the N-terminus of an epitope to be internalized by an antigen presenting cell so that the N-term sequence of the extended epitope is X1-X2-X3 or X1-X2-X3-X4, wherein X1, X2, X3 and X4 are as defined in any of claims 1 to 5; for improving presentation of the epitope by the antigen presenting cells, in comparison with non-extended epitope.
